# EUROPÄISCHE PATENTSCHRIFT

(11) **EP 3 417 906 B1**
(45) Veröffentlichungstag und Bekanntmachung des Hinweises auf die Patenterteilung: **09.03.2022**
(21) Anmeldenummer: 18176767.4
(22) Anmeldetag: 08.06.2018
(51) Int. Cl.: A61M 39/22, A61B 1/00, A61M 1/00, A61B 1/015, F16K 3/24, F16K 27/04, A61M 5/168, F16K 11/07, F16K 31/524, F16K 31/528

(54) **MEDIZINISCHES DRUCKTASTENVENTIL**
MEDICAL PUSH KEY VALVE
VANNE À BOUTON POUSSOIR MÉDICAL

(30) Priorität: 16.06.2017 DE 102017005718
(43) Veröffentlichungstag der Anmeldung: 26.12.2018
(73) Patentinhaber: Karl Storz SE & Co. KG, 78532 Tuttlingen (DE)
(72) Erfinder: Tobien, Johannes Peter, 78532 Tuttlingen (DE)
(74) Vertreter: Hofmeister, Frank

(56) Entgegenhaltungen:
- EP-A1- 1 707 107
- EP-A1- 2 997 876
- JP-A- H1 052 399
- US-A- 5 427 144

## Beschreibung

Die Erfindung betrifft ein medizinisches Drucktastenventil mit einem Ventilgehäuse, einem in dem Ventilgehäuse ausgebildeten Strömungskanal sowie mit einem Ventilkolben, der zwischen einer den Strömungskanal freigebenden und einer den Strömungskanal verschließenden Position verlagerbar im Ventilgehäuse angeordnet ist, wobei der Ventilkolben mit einem am Ventilgehäuse gelagerten, über mindestens ein Federelement federbelastetes Druckelement in Wirkverbindung steht und der Ventilkolben über das federbelastete Druckelement in die den Strömungskanal verschließende Position vorgespannt ist und das federbelastete Druckelement in mindestens einer Position arretierbar ist, in der der mit dem Druckelement in Wirkverbindung stehende Ventilkolben den Strömungskanal zumindest teilweise freigibt.

Während chirurgischer Operationen ist es häufig erforderlich, Flüssigkeiten, wie beispielsweise Blut oder Spülflüssigkeit, aus dem Operationsgebiet abzusaugen. Hierzu können mit einem Saugkanal ausgestattete chirurgische Instrumente über eine vorzugsweise flexible Saugleitung mit einer externen Saugquelle, beispielsweise einer Vakuumpumpe, verbunden werden.

Da in Operationssälen die Leistung der den Saugstrom erzeugenden Vakuumpumpe in der Regel nicht regulierbar ist, ist es üblich, die medizinischen Instrumente, wie beispielsweise Endoskope, mit einem Ventil auszustatten, über das der Saugstrom zu- und abschaltbar ist.

Aus der Praxis ist es bekannt, ein solches Ventil zum Zu- und/oder Abschalten des Saugstroms als Drucktastenventil auszubilden, bei dem der den Strömungskanal freigebende oder verschließende Ventilkörper über ein federbelastetes Druckelement betätigbar ist, wobei der Ventilkörper über ein Federelement in die den Strömungskanal verschließende Position vorgespannt ist.

Diese bekannten Drucktastenventile, bei denen der Operateur zum Freigeben des Strömungskanals das federbelastete Druckelement eindrücken muss, haben sich in der Praxis bewährt. Nachteilig an dieser bekannten Konstruktion des Drucktastenventils ist, dass der Operateur das federbelastete Druckelement während des gesamten Saugvorgangs gedrückt halten muss, da der Ventilkörper in die den Saugkanal schließende Position vorgespannt ist. Derartige Ventile sind z.B. aus der EP 2 997 876, EP 1 707 107 und US 5 427 144 bekannt.

Bei manchen Untersuchungen und/oder Behandlungen, wie beispielsweise des Gallenganges, ist es erforderlich, eine kontinuierliche Absaugung zu gewährleisten, um ein Austreten von Sekreten aus einem anderen Arbeitskanalzugang zu verhindern. Das dauerhafte Drücken des federbelasteten Druckelements zur Gewährleistung der kontinuierlichen Absaugung ist für den Operateur anstrengend und kann aufgrund der gleichbleibenden Halteposition zum Verkrampfen des Fingers führen.

Ein gattungsgemäßes Ventil ist beispielsweise aus der JP-H10-52399 A bekannt. Bei diesem bekannten Ventil ist der Ventilkolben zur Überführung in eine den Strömungskanal freigebende Position über einen federbelasteten Drücker nach unten drückbar. Um das Ventil in der den Strömungskanal freigebenden Position arretieren zu können, ist am Drücker ein angeformter Vorsprung vorgesehen, der durch Verdrehen des Drückers in eine Nut überführbar ist, so dass die Rückstellkraft des Federelements den Drücker nicht mehr zurück in die den Strömungskanal verschließende Position überführen kann.

Durch die Ausbildung der Arretierposition des Druckelements in einer Position, in der der Ventilkörper den Strömungskanal freigibt, wird der Operateur von dem dauerhaften Niederdrücken des Druckelements befreit.

Davon ausgehend liegt der Erfindung die **Aufgabe** zugrunde, ein weiteres medizinisches Drucktastenventil zu schaffen, das eine einfache und ermüdungsfreie Einhandbedienung ermöglicht.

Die **Lösung** dieser Aufgabenstellung ist erfindungsgemäß dadurch gekennzeichnet, dass in Richtung der Längsachse des Ventilgehäuses betrachtet am Ventilkolben mit Abstand zueinander zwei Verschlusskörper ausgebildet sind, die mit entsprechenden Ventilsitzen zusammenwirken.

Während der eine Verschlusskörper mit seinem zugehörigen Ventilsitzen zum Abdichten des Drückergehäuses gegenüber der Umgebungsluft dient lässt sich über den anderen Verschlusskörper mit seinem zugehörigen Ventilsitz der Strömungskanal patientenseitig verschließen.

Die Ausbildung der zwei Verschlusskörper an dem gemeinsamen Ventilkolben stellt sicher, dass das Öffnen und Verschließen des Strömungskanals und das Abdichten und Öffnen des Drückergehäuses gegenüber der Umgebungsluft zwangsweise parallel zueinander erfolgt.

Die Verschlusskörper des Ventilkolbens sind erfindungsgemäß als Verdickungen des Ventilkolbens mit konischen Übergangsbereichen ausgebildet sind. Die konische Ausbildung der Verschlusskörper verhindert, dass der Strömungskanal beim Verstellen des Ventilkolbens schlagartig geschlossen oder geöffnet wird. Aufgrund der Konizität der Verschlusskörper ist ein langsames Öffnen und Schließen des Strömungskanals möglich.

Gemäß einer bevorzugten Ausführungsform der Erfindung ist das federbelastete Druckelement ausschließlich in der Position arretierbar, in der der mit dem Druckelement in Wirkverbindung stehende Ventilkolben den Strömungskanal vollständig freigibt. In dieser arretierten Position des Druckelements bzw. des mit diesem in Wirkverbindung stehenden Ventilkolbens liegt die volle Saugleistung am zum Patienten führenden distalen Ende des Ventilgehäuses an.

Das federbelastete Druckelement ist in einem am proximalen Ende des Ventilgehäuses angeordneten Drückergehäuse gelagert, wobei das federbelastete Druckelement relativ zum Drückergehäuse um die Längsachse des Ventilgehäuses verdrehbar ist.

Zur Ausbildung der Arretierfunktion des federbelasteten Druckelements sind das federbelastete Druckelement und das Drückergehäuse über eine Zapfen-Schlitz-Steuerung miteinander gekoppelt.

Die Zapfen-Schlitz-Steuerung ermöglicht eine geführte Verlagerung eines an einem Bauteil ausgebildeten Zapfens in einer Art Kulisse, die am anderen Bauteil ausgebildet ist, wobei für die Funktionsweise unerheblich ist, an welchem der Bauteile Druckelement oder Drückergehäuse der Zapfen oder die Führung für den Zapfen ausgebildet ist.

Die Zapfen-Schlitz-Steuerung ist als Nut und Rastfläche ausgebildet, wobei die am verdrehbaren Druckelement ausgebildete Nut die am Drückergehäuse ausgebildete Rastfläche in der Arretierposition in sich aufnimmt. Die Rastfläche ist an der Innenseite des Drückergehäuses angeordnet und als eine sich halbkreisförmig radial nach innen erstreckende Fläche ausgebildet.

Zur Ausbildung des auf das Druckelement wirkenden Federelements wird mit der Erfindung vorgeschlagen, dass das mindestens eine Federelement, über das Druckelement federbelastet ist, einstückig mit dem Druckelement oder dem Drückergehäuse ausgebildet ist, wobei die Federelastizität des Materials, aus dem das Druckelement bzw. das Drückergehäuse gefertigt sind, zur Ausbildung der Federeigenschaft herangezogen wird.

Gemäß einer praktischen Ausführungsform der Erfindung wird vorgeschlagen, dass im Übergangsbereich vom Ventilgehäuse zum Drückergehäuse auf der Außenseite der beiden Gehäuse und ein die beiden Gehäuse miteinander verbindender elastischer Klemmring angeordnet ist. Diese Art der Verbindung der beiden Gehäuse über den elastischen Klemmring stellt eine besonders einfache und schnell durchzuführende Art der Montage und Demontage dar.

Um eine besonders einfache Bedienung des federbelasteten Druckelements mit nur einer Hand oder auch nur einem Finger zu ermöglichen, wird mit der Erfindung vorgeschlagen, dass am proximalen Ende des federbelasteten Druckelements ein radial nach außen abstehender Betätigungshebel angeordnet ist. Über den Betätigungshebel lässt sich das federbelastete Druckelement einfach verdrehen, um die Arretierung über die Zapfen-Schlitz-Steuerung zu bewerkstelligen.

Schließlich wird mit der Erfindung vorgeschlagen, dass in der Außenkontur des proximalen Endes des federbelasteten Druckelements Griffmulden ausgebildet sind.

Weitere Merkmale und Vorteile der Erfindung ergeben sich anhand der zugehörigen Zeichnungen, in denen zwei Ausführungsbeispiele eines erfindungsgemäßen medizinischen Drucktastenventils nur beispielhaft dargestellt sind, ohne die Erfindung auf diese Ausführungsbeispiele zu beschränken. In den Zeichnungen zeigt:
- Fig. 1a: eine teilweise freigeschnittene Seitenansicht einer ersten Ausführungsform eines Drucktastenventils, das Ventil in der den Strömungskanal verschließenden Position darstellend;
- Fig. 1b: einen Längsschnitt entlang der Linie Ib-Ib gemäß Fig. 1a;
- Fig. 2a: eine Darstellung gemäß Fig. 1a, jedoch das Ventil in einer den Strömungskanal freigebenden Position darstellend;
- Fig. 2b: einen Längsschnitt entlang der Linie IIb-IIb gemäß Fig. 2a;
- Fig. 3a: eine Darstellung gemäß Fig. 2a, jedoch das Druckelement zusätzlich in einer arretierten Position darstellend;
- Fig. 3b: einen Längsschnitt entlang der Linie IIIb-IIIb gemäß Fig. 3a;
- Fig. 4: eine perspektivische Ansicht des vom Ventilgehäuse demontierten Drückergehäuses einschließlich Ventilkolben;
- Fig. 5a: eine teilweise freigeschnittene Seitenansicht einer zweiten Ausführungsform eines erfindungsgemäßen Drucktastenventils, das Ventil in der den Strömungskanal verschließenden Position darstellend;
- Fig. 5b: einen Längsschnitt entlang der Linie Vb-Vb gemäß Fig. 5a;
- Fig. 5c: einen Querschnitt entlang der Linie Vc-Vc gemäß Fig. 5a;
- Fig. 6a: eine Darstellung gemäß Fig. 5a, jedoch das Ventil in einer den Strömungskanal freigebenden Position darstellend;
- Fig. 6b: einen Längsschnitt entlang der Linie VIb-VIb gemäß Fig. 6a;
- Fig. 6c: einen Querschnitt entlang der Linie VIc-VIc gemäß Fig. 6a
- Fig. 7a: eine Darstellung gemäß Fig. 6a, jedoch das Druckelement zusätzlich in einer fast vollständig arretierten Position darstellend;
- Fig. 7b: einen Längsschnitt entlang der Linie Vllb-Vllb gemäß Fig. 7a;
- Fig. 7c: einen Querschnitt entlang der Linie Vllc-Vllc gemäß Fig. 7a
- Fig. 8a: eine Darstellung gemäß Fig. 6a, jedoch das Druckelement zusätzlich in einer vollständig arretierten Position darstellend;
- Fig. 8b: einen Längsschnitt entlang der Linie Vlllb-Vlllb gemäß Fig. 8a und
- Fig. 8c: einen Querschnitt entlang der Linie VIIIc-VIIIc gemäß Fig. 8a

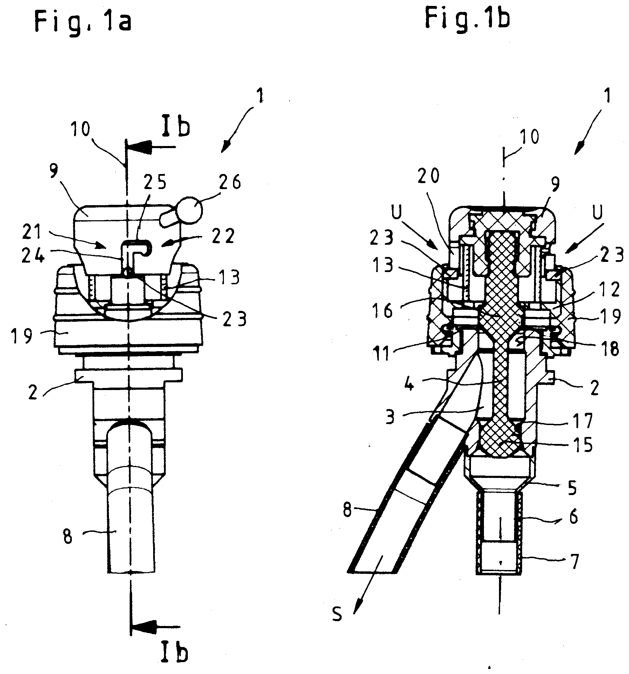

Die Abbildung Fig. 1a bis 3b sowie Fig. 5a bis 8c zeigen ein medizinisches Drucktastenventils 1, das im Wesentlichen aus einem Ventilgehäuse 2, einem in dem Ventilgehäuse 2 ausgebildeten Strömungskanal 3 sowie einem Ventilkolben 4 besteht, der zwischen einer den Strömungskanal 3 freigebenden und einer den Strömungskanal 3 verschließenden Position verlagerbar im Ventilgehäuse 2 angeordnet ist.

Wie aus der geschnittenen Seitenansichten gemäß den Abbildungen Fig. 1b, 2b und 3b ersichtlich, ist am distalen Ende 5 des Ventilgehäuses 2 ein Anschlussstutzen 6 angeordnet, über den das Drucktastenventil 1 an eine zum Patienten führende Leitung 7 anschließbar ist.

Mit der von einer externen Saugquelle stammenden Saugleistung wird das Drucktastenventil 1 über eine Saugleitung 8 beaufschlagt, die unter einem spitzen Winkel seitlich in das Ventilgehäuse 2 mündet.

Das Verstellen des Ventilkolbens 4 zwischen der den Strömungskanal 3 freigebenden und der den Strömungskanal 3 verschließenden Position erfolgt über ein Druckelement 9, das in Richtung der Längsachse 10 des Ventilgehäuses 2 axial verlagerbar in einem am proximalen Ende 11 des Ventilgehäuses 2 angeordneten Drückergehäuse 12 gelagert ist. Weiterhin ist das Druckelement 9 relativ zum Drückergehäuse 12 um die Längsachse 10 des Ventilgehäuses 2 verdrehbar.

Das Zusammenwirken zwischen dem Druckelement 9 und dem Ventilkolben 4 ist dabei so ausgelegt, dass der Ventilkolben 4 über ein auf das Druckelement 9 wirkendes Federelement 13 in die den Strömungskanal 3 verschließende Position vorgespannt ist. Dies bedeutet, dass zum Öffnen des Strömungskanals 3 das Druckelement 9 gegen die Kraft des Federelements 13 in Richtung der Längsachse 10 des Ventilgehäuses 2 nach distal gedrückt werden muss, um den Ventilkolben 4 in die den Strömungskanal 3 freigebende Position zu überführen.

Alternativ zu der dargestellten Ausbildung des Federelements 13 als wendelförmige Druckfeder ist es auch möglich, das Federelement, über das Druckelement 9 federbelastet ist, einstückig mit dem Druckelement 9 oder mit dem Drückergehäuse 12 auszubilden, wobei die Federelastizität des Materials, aus dem das Druckelement 9 bzw. das Drückergehäuse 12 gefertigt sind, zur Ausbildung der Federeigenschaft herangezogen wird.

Wie beispielsweise aus der Abbildung Fig. 1b ersichtlich, sind in Richtung der Längsachse 10 des Ventilgehäuses 2 betrachtet am Ventilkolben 4 mit Abstand zueinander zwei Verschlusskörper 15 und 16 ausgebildet sind, die mit entsprechenden Ventilsitzen 17 und 18 zusammenwirken. Die Funktionsweise der beiden Verschlusskörper 15 und 16 sowie der beiden Ventilsitze 17 und 18 wird nachfolgend bei der Beschreibung der Funktionsweise des Drucktastenventils 1 beschrieben.

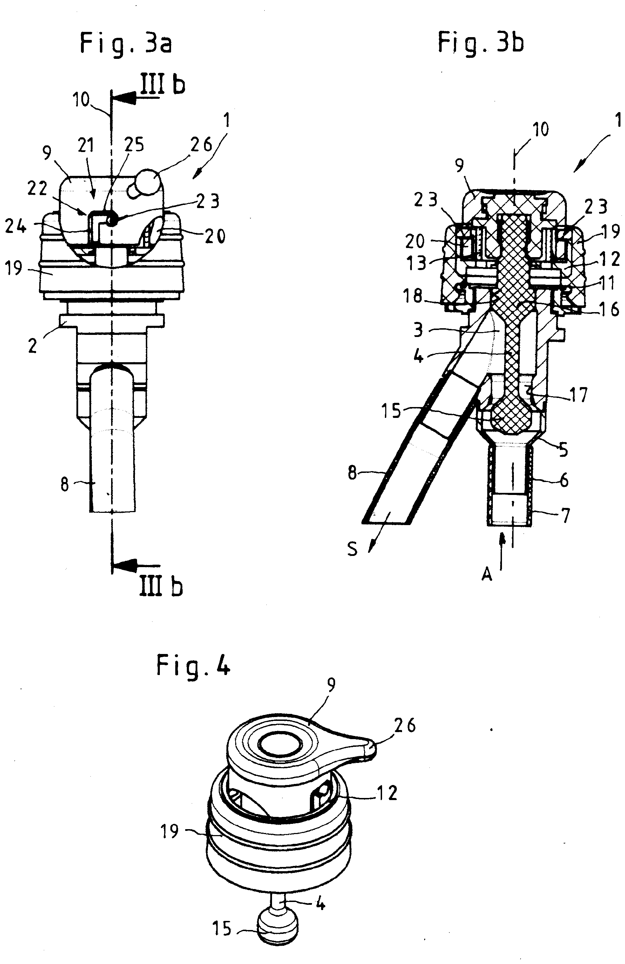

Im Übergangsbereich vom Ventilgehäuse 2 zum Drückergehäuse 12 ist auf der Außenseite der beiden Gehäuse 2 und 12 ein elastischer Klemmring 19 angeordnet, der am Drückergehäuse 12 festegelegt dazu dient, das Drückergehäuse 12 mit dem Ventilgehäuse 2 zu verbinden. Die Abbildung Fig. 4 zeigt das Drückergehäuse 12 vom Ventilgehäuse 2 demontiert. Zur Montage mit dem Ventilgehäuse 2 wird der mit dem Drückergehäuse 12 verbundene elastische Klemmring 19 über den oberen Teil des Ventilgehäuses 2 gestülpt und verbindet so die beiden Gehäuse 2 und 12 miteinander.

Zu Reinigungszwecken lassen sich vorteilhafterweise das Drückergehäuse 12 und das Ventilgehäuse 2 voneinander trennen, wobei der Ventilkolben 4 zusammen mit dem Drückergehäuse 12 nach proximal aus dem Ventilgehäuse 2 ziehbar ist, wie dies in Fig. 4 dargestellt ist.

Die Arbeitsweise des medizinischen Drucktastenventils 1 wird nachfolgend anhand der Abbildungen Fig. 1a bis 3b beschrieben.

Die in Fig. 1b gezeigte Schnittdarstellung des Drucktastenventils 1 zeigt die unbetätigte Ausgangsposition des Drucktastenventils 1, in der der Ventilkolben 4 den im Ventilgehäuse 2 ausgebildeten Strömungskanal 3 distalseitig vollständig verschließt. In dieser geschlossenen Position liegt der an dem dem Druckelement 9 fernen Ende des Ventilkolbens 4 angeordnete Verschlusskörper 15 radial umfänglich abdichtend an dem im Ventilgehäuse 2 ausgebildeten Ventilsitz 17 an und verschließt so den Strömungskanal 3 im Wesentlichen fluiddicht.

Über das sich einseitig am Drückergehäuse 12 abstützende Federelement 13 wird das Druckelement 9, das bei der in den Abbildungen Fig. 1a bis 3b dargestellten ersten Ausführungsform zweiteilig ausgebildet ist, in Richtung der Längsachse 10 des Ventilgehäuses 2 nach proximal (in der Abbildung Fig. 1b nach oben) gedrückt.

Da in Operationssälen die Leistung der den Saugstrom erzeugenden Vakuumpumpe in der Regel nicht regulierbar ist, also auch in geschlossenen Position des Ventilkolbens 4 über die Saugleitung 8 ein dauerhafter Saugstrom in Richtung des Pfeils S am Drucktastenventil 1 anliegt, sind im Drückergehäuse 12 Zuluftöffnungen 20 ausgebildet, über die Umgebungsluft in Richtung des Pfeils U angesaugt und über die Saugleitung 8 abgeführt werden kann. Durch die über die Zuluftöffnungen 20 ansaugbare Umgebungsluft U wird verhindert, dass aufgrund der dauerhaften Saugleistung über die Saugleitung 8 ein Unterdruck im Ventilgehäuse 2 entsteht, der einerseits Dichtelemente im Drucktastenventil 1 stark beanspruchen würde und andererseits das Betätigen des Ventilkolbens 4 erschweren könnte.

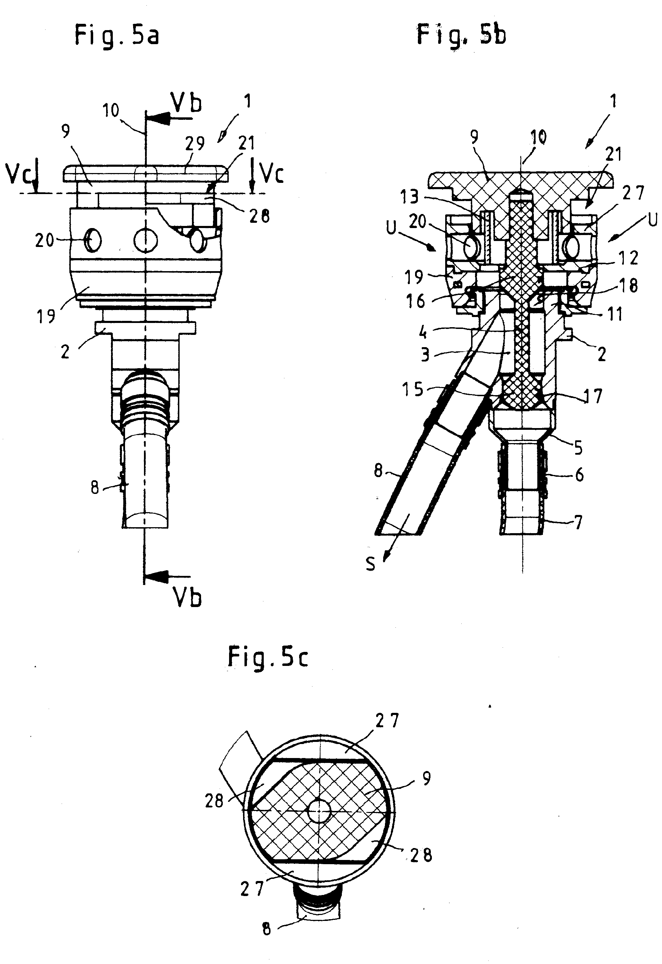

Wie insbesondere aus den Abbildungen Fig. 1a, 2a und 3a ersichtlich, sind das federbelastete Druckelement 9 und das Drückergehäuse 12 über eine Zapfen-Schlitz-Steuerung 21 miteinander gekoppelt, die bei der dargestellten ersten Ausführungsform als Bajonettverschluss 22 mit einem Raststift 23 und einer Kulissenführung 24 ausgebildet ist. Der hier dargestellte Bajonettverschluss 22 ist nicht Teil der Erfindung. Erfindungsgemäß wird das Druckelement 9 über eine an diesem vorgesehene Nut 28 mit einer Rastfläche 28 des Drückergehäuses 12 gekoppelt, wie in den Fig.5-8 dargestellt und weiter unten beschrieben.

Die dargestellte Zapfen-Schlitz-Steuerung 21 besteht aus zwei über den Umfang des Drückergehäuses 12 um 180° Grad zueinander versetzt angeordneten Bajonettverschlüssen 22. Den Abbildungen Fig. 1b, 2b und 3b sind die zwei Raststifte 23 der Bajonettverschlüsse 22 zu entnehmen. Durch die Verwendung von zwei Bajonettverschlüssen 22 wird ein verkantungsfreies Betätigen der Zapfen-Schlitz-Steuerung 21 gewährleistet.

Alternativ zu der Verwendung von zwei um 180° zueinander versetzt angeordneten Bajonettverschlüssen 22 können je nach Umfang des Drückergehäuses 12 auch mehr als zwei Bajonettverschlüsse 22, wie beispielsweise drei um jeweils 120° zueinander versetzt angeordnete Bajonettverschlüsse 22, verwendet werden.

Bei der in Fig. 1a dargestellten Position des Drucktastenventils 1, bei der sich der Ventilkolben 4 in der den Strömungskanal 3 verschließenden Position befindet, ist der Raststift 23 am unteren Ende der Kulissenführung 24 angeordnet.

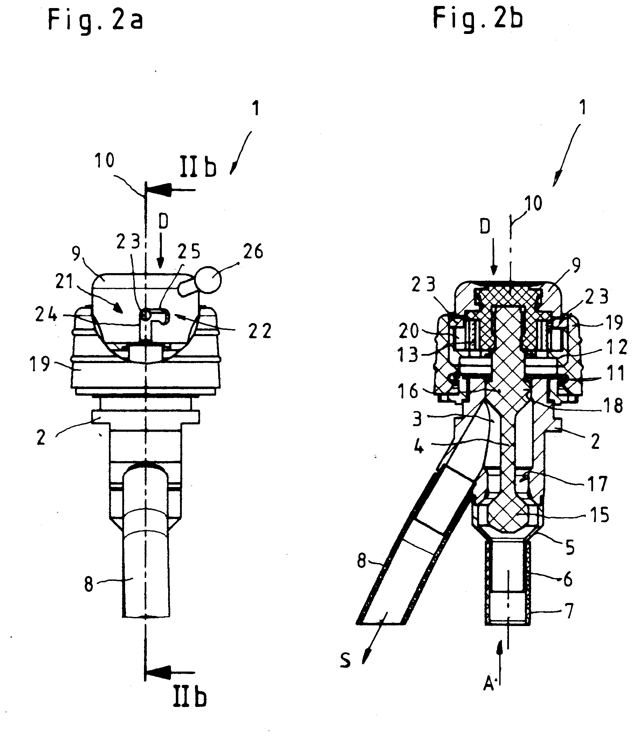

Die in Fig. 2b gezeigte Schnittdarstellung des Drucktastenventils 1 zeigt die betätigte Position des Drucktastenventils 1, in der der Ventilkolben 4 den im Ventilgehäuse 2 ausgebildeten Strömungskanal 3 distalseitig vollständig freigibt. In dieser geöffneten Position liegt der Verschlusskörper 15 des Ventilkolbens 4 nicht mehr abdichtend an dem im Ventilgehäuse 2 ausgebildeten Ventilsitz 17 an, sondern ist in Richtung der Längsachse 10 des Ventilgehäuses 2 fort vom Ventilsitz 17 nach distal (in der Abbildung Fig. 2b nach unten) verlagert.

Das Verstellen des Ventilkolbens 4 von der in Fig. 1b dargestellten, den Strömungskanal 3 verschließenden Position in die in Fig. 2b dargestellte, den Strömungskanal 3 freigebenden Position erfolgt durch Eindrücken des federbelasteten Druckelements 9 in Richtung der Längsachse 10 des Ventilgehäuses 2 nach distal (in der Abbildung Fig. 2b nach unten) durch eine Druckkraft D. Die axiale Druckbewegung des Druckelements 9 wird spielfrei auf den Ventilkolben 4 übertragen, wodurch der Verschlusskörper 15 aus der abdichtenden Anlage am Ventilsitz 17 gedrückt wird.

In dieser geöffneten Position des Strömungskanals 3 wird der Zustrom der Umgebungsluft U über die Zuluftöffnungen 20 durch den zweiten, dem Druckelement 9 nahe angeordneten Verschlusskörper 16 des Ventilkolbens 4 verschlossen, da dieser Verschlusskörper 16 bei herabgedrücktem Druckelement 9 radial umfänglich abdichtend an dem korrespondierenden zweiten im Ventilgehäuse 2 ausgebildeten Ventilsitz 18 anliegt.

Das Verschließen des in Fig. 1b dargestellten Zustroms der Umgebungsluft U bewirkt, dass nunmehr die volle Saugleistung der Saugleitung 8 am Strömungskanal 3 anliegt und über die Leitung 7 Flüssigkeit vom Patienten her in Richtung der durch den Pfeil gekennzeichneten Absaugrichtung A abgesaugt werden kann.

Bei der in Fig. 2a dargestellten Position des Drucktastenventils 1, bei der sich der Ventilkolben 4 in der den Strömungskanal 3 freigebenden Position befindet, ist der Raststift 23 am oberen senkrechten Ende der Kulissenführung 24 angeordnet.

Da das Federelement 13 so angeordnet ist, dass das Druckelement 9 und somit auch der Ventilkolben 4 in die den Strömungskanal 3 verschließende Position vorgespannt ist, muss der Operateur das Druckelement 9 mit der Druckkraft D nach unten gedrückt halten, so lange der Saugdruck an der Leitung 7 anliegen soll.

Um den Operateur zu entlasten, ist es gemäß der in den Abbildungen Fig. 3a und 3b dargestellten Position des Drucktastenventils 1 möglich, das Druckelement 9 und somit auch den mit dem Druckelement 9 in Wirkverbindung stehenden Ventilkolben 4 in der den Strömungskanal 3 freigebenden Position zu arretieren.

Zu diesem Zweck weist die Kulissenführung 24 an ihren oberen Ende eine rechtwinklige Abwinklung 25 auf. Da das Druckelement 9 relativ zum Drückergehäuse 12 um die Längsachse 10 des Ventilgehäuses 2 verdrehbar ist, lässt sich der Raststift 23 durch Verdrehen des Druckelements 9 aus der in Fig. 2a dargestellten Position einfach in die in Fig. 3a dargestellte Position in der Abwinklung 25 überführen. In dieser Lage des Raststiftes 23 in der Abwinklung 25 kann das Federelement 13 das Druckelement 9 auch ohne die der Federkraft des Federelements 13 entgegenwirkende Druckkraft D nicht wieder nach oben zurück in die den Strömungskanal 3 verschließende Position drücken.

Um das Verdrehen des Druckelements 9 zum Betätigen des Bajonettverschlusses 22 zu erleichtern und auch eine Betätigung mit nur einem Finger zu ermöglichen, ist am proximalen Ende des federbelasteten Druckelements 9 ein radial nach außen abstehender Betätigungshebel 26 angeordnet. Die Abbildung Fig. 4 zeigt eine gegenüber den Abbildungen Fig. 1a, 2a und 3a abgewandelte Ausführungsform des Betätigungshebels 26.

Zum erneuten Verschließen des Strömungskanals 3 durch den Ventilkolben 4 muss der Operateur nur das Druckelement 9 wieder um die Längsachse 10 des Ventilgehäuses 2 verdrehen, bis der Raststift 23 wieder außer Eingriff mit der Abwinklung 25 tritt, wie dies in Fig. 2a dargestellt ist. Sobald der Operateur dann keine Druckkraft D mehr auf das Druckelement 9 ausübt, drückt das Federelement 13 das Druckelement 9 und somit auch den mit dem Druckelement 9 in Wirkverbindung stehenden Ventilkolben 4 zurück in die den Strömungskanal 3 verschließende Position gemäß Fig. 1b. In dieser Position liegt einerseits der Verschlusskörper 15 wieder den Strömungskanal 3 abdichtend am Ventilsitz 17 an und liegt andererseits der Verschlusskörper 16 nicht mehr am Ventilsitz 18 an, so dass wieder Umgebungsluft U über die Zuluftöffnungen 20 angesaugt werden kann.

Die Zapfen-Schlitz-Steuerung 21 ermöglicht bei der Betätigung des Druckelements 9 eine geführte Verlagerung des Raststiftes 23 in der Kulissenführung 24.

Auch wenn in den Abbildungen Fig. 3a und 3b die arretierte Position des Ventilkolbens 4 so dargestellt ist, dass dieser den Strömungskanal 3 vollständig freigibt, ist es selbstverständlich auch möglich, die Arretierung des Druckelements 9 bzw. des Ventilkolbens 4 in jeder Zwischenposition zwischen der den Strömungskanal 3 verschließenden und der den Strömungskanal 3 freigebenden Position vorzunehmen oder aber auch unterschiedliche Arretierpositionen an einem Drucktastenventil 1 zu ermöglichen.

Wie weiterhin aus den Abbildungen ersichtlich, sind die Verschlusskörper 15 und 16 des Ventilkolbens 4 als Verdickungen des Ventilkolbens 4 mit konischen Übergangsbereichen ausgebildet. Die konische Ausbildung der Übergangsbereiche vom Ventilkolben 4 zum eigentlichen Verschlusskörper 15 und 16 verhindert, dass der Strömungskanal 3 beim Verstellen des Ventilkolbens 4 schlagartig geschlossen oder geöffnet wird. Aufgrund der Konizität der Verschlusskörper 15 und 16 ist ein langsames Öffnen und Schließen des Strömungskanals 3 möglich.

Die in Fig. 5a bis 8c dargestellte zweite Ausführungsform der Erfindung zur Ausbildung des Drucktastenventils 1 unterscheidet sich von der zuvor dargestellten Ausführungsform dadurch, dass die Zapfen-Schlitz-Steuerung 21 als Rastfläche 27 und auf die Rastfläche 27 aufschiebbare Nut 28 ausgebildet ist.

Die Nut 28 ist dabei am Druckelement 9 ausgebildet ist und die Rastfläche 27 als an der Innenseite des Drückergehäuses 12 angeordnete, sich halbkreisförmig radial nach innen erstreckende Fläche ausgebildet.

Die in Fig. 5b gezeigte Schnittdarstellung des Drucktastenventils 1 zeigt die unbetätigte Ausgangsposition des Drucktastenventils 1, in der der Ventilkolben 4 den im Ventilgehäuse 2 ausgebildeten Strömungskanal 3 distalseitig vollständig verschließt. In dieser geschlossenen Position liegt der an dem dem Druckelement 9 fernen Ende des Ventilkolbens 4 angeordnete Verschlusskörper 15 radial umfänglich abdichtend an dem im Ventilgehäuse 2 ausgebildeten Ventilsitz 17 an und verschließt so den Strömungskanal 3 im Wesentlichen fluiddicht.

Auch bei dieser Ausführungsform sind im Drückergehäuse 12 Zuluftöffnungen 20 ausgebildet, über die Umgebungsluft in Richtung des Pfeils U angesaugt und über die Saugleitung 8 abgeführt werden kann, wenn der Strömungskanal 3 distalseitig über den Ventilkolben 4 verschlossen ist.

Wie insbesondere aus den Abbildungen Fig. 5c, 6c, 7c und 8c ersichtlich, ist die Zapfen-Schlitz-Steuerung 21, über die das Druckelement 9 und das Drückergehäuse 12 miteinander gekoppelt sind, als Rastfläche 27 und auf die Rastfläche 27 aufschiebbare Nut 28 ausgebildet.

Bei der in Fig. 5a und 5b dargestellten Position des Drucktastenventils 1, bei der sich der Ventilkolben 4 in der den Strömungskanal 3 verschließenden Position befindet, ist die am Druckelement 9 ausgebildete Nut 28 oberhalb der Rastfläche 27 angeordnet.

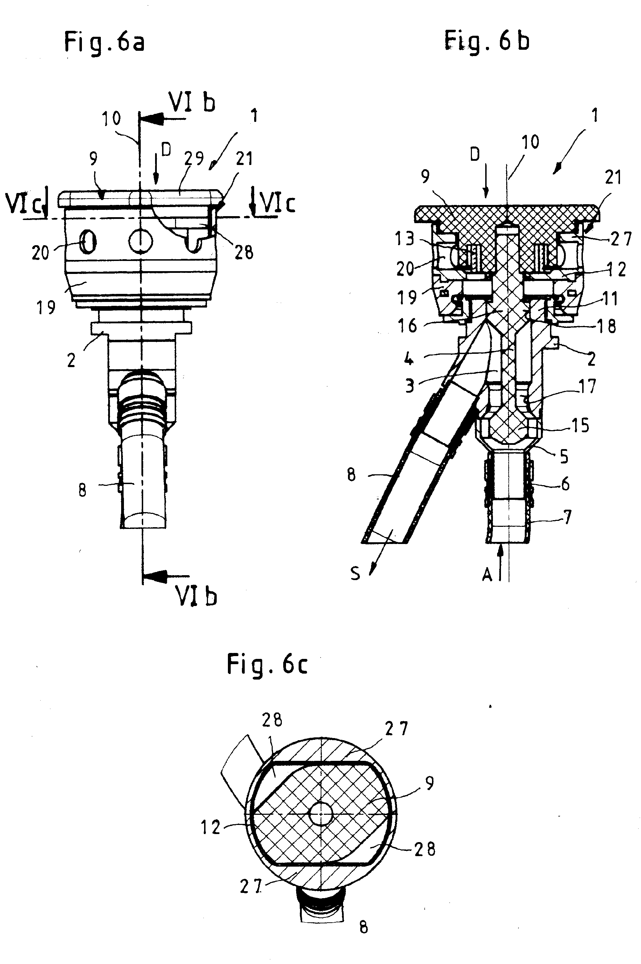

Die in Fig. 6b gezeigte Schnittdarstellung des Drucktastenventils 1 zeigt die betätigte Position des Drucktastenventils 1, in der der Ventilkolben 4 den im Ventilgehäuse 2 ausgebildeten Strömungskanal 3 distalseitig vollständig freigibt. In dieser geöffneten Position liegt der Verschlusskörper 15 des Ventilkolbens 4 nicht mehr abdichtend an dem im Ventilgehäuse 2 ausgebildeten Ventilsitz 17 an, sondern ist in Richtung der Längsachse 10 des Ventilgehäuses 2 fort vom Ventilsitz 17 nach distal (in der Abbildung Fig. 6b nach unten) verlagert.

Das Verstellen des Ventilkolbens 4 von der in Fig. 5b dargestellten, den Strömungskanal 3 verschließenden Position in die in Fig. 6b dargestellten, den Strömungskanal 3 freigebenden Position erfolgt durch Eindrücken des federbelasteten Druckelements 9 in Richtung der Längsachse 10 des Ventilgehäuses 2 nach distal (in der Abbildung Fig. 6b nach unten) durch eine Druckkraft D. Die axiale Druckbewegung des Druckelements 9 wird spielfrei auf den Ventilkolben 4 übertragen, wodurch der Verschlusskörper 15 aus der abdichtenden Anlage am Ventilsitz 17 gedrückt wird.

In dieser geöffneten Position des Strömungskanals 3 wird der Zustrom der Umgebungsluft U über die Zuluftöffnungen 20 durch den zweiten, dem Druckelement 9 nahe angeordneten Verschlusskörper 16 des Ventilkolbens 4 verschlossen, da dieser Verschlusskörper 16 bei herabgedrücktem Druckelement 9 radial umfänglich abdichtend an dem korrespondierenden zweiten im Ventilgehäuse 2 ausgebildeten Ventilsitz 18 anliegt.

Das Verschließen des Zustroms der Umgebungsluft U bewirkt, dass nunmehr die volle Saugleistung der Saugleitung 8 am Strömungskanal 3 anliegt und über die Leitung 7 Flüssigkeit vom Patienten her in Richtung der durch den Pfeil gekennzeichneten Absaugrichtung A abgesaugt werden kann.

Bei der in Fig. 6a dargestellten Position des Drucktastenventils 1, bei der sich der Ventilkolben 4 in der den Strömungskanal 3 freigebenden Position befindet, liegt die am Druckelement 9 ausgebildete Nut 28 in einer Ebene mit der auf der Innenseite des Drückergehäuses 12 angeordneten Rastfläche 27.

Da das Federelement 13 so angeordnet ist, dass das Druckelement 9 und somit auch der Ventilkolben 4 in die den Strömungskanal 3 verschließende Position vorgespannt ist, muss der Operateur das Druckelement 9 mit der Druckkraft D nach unten gedrückt halten, so lange der Saugdruck an der Leitung 7 anliegen soll.

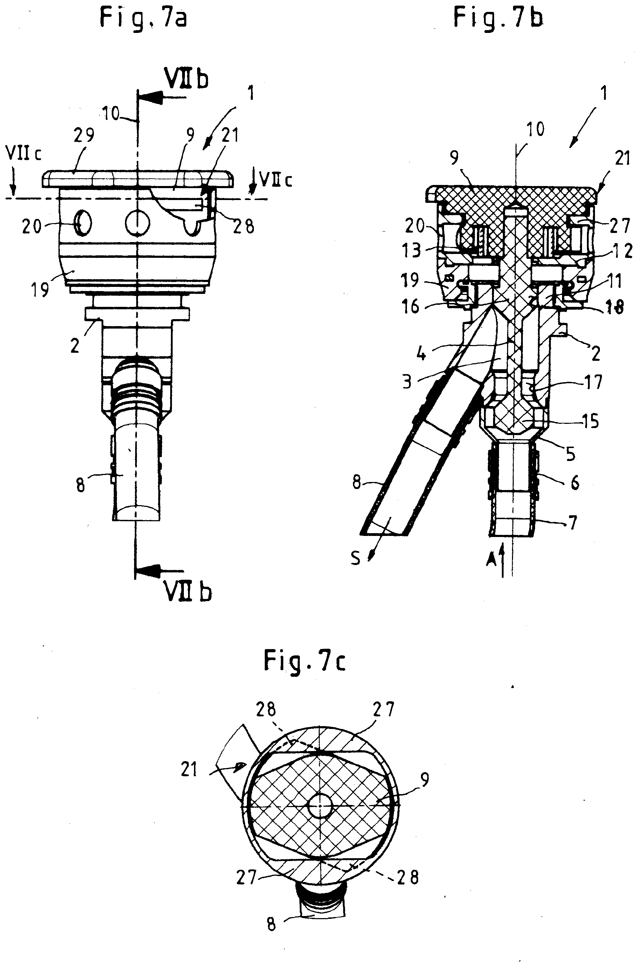

Um den Operateur zu entlasten, ist es gemäß der in den Abbildungen Fig. 7a bis 8c dargestellten Positionen des Drucktastenventils 1 möglich, das Druckelement 9 und somit auch den Ventilkolben 4 in der den Strömungskanal 3 freigebenden Position zu arretieren.

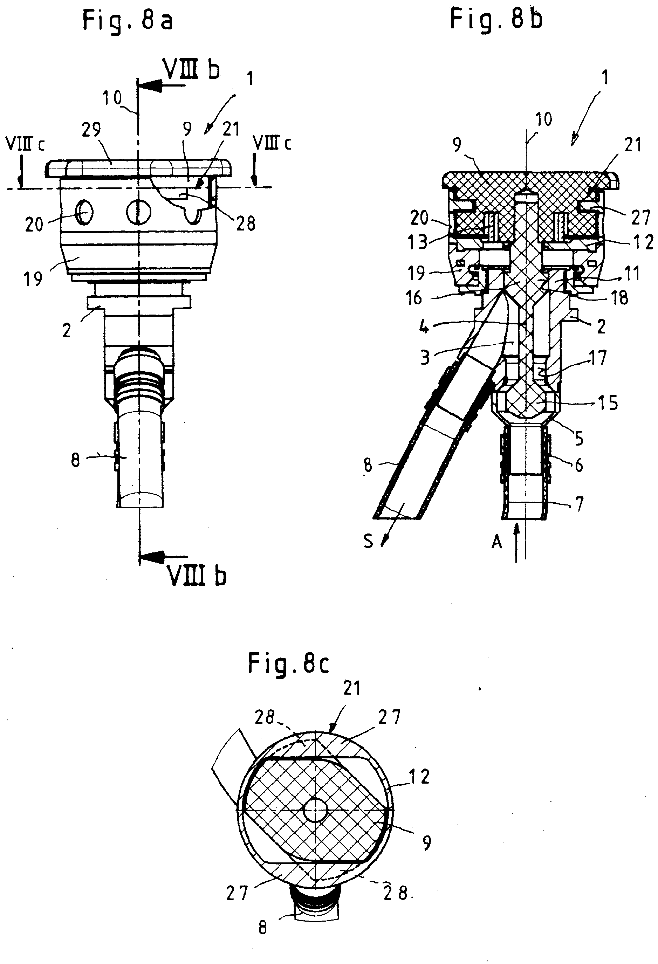

Zu diesem Zweck lässt durch Verdrehen des Druckelements 9 um die Längsachse 10 des Ventilgehäuses 2 die am Druckelement 9 ausgebildete Nut 28 in die in den Abbildungen Fig. 7c und 8c dargestellten Positionen überführen, in der die Rastfläche 27 des Drückergehäuses 12 arretierend in der Nut 28 aufgenommen wird. In dieser Lage der Rastfläche 27 in der Nut 28 kann das Federelement 13 das Druckelement 9 auch ohne die der Federkraft des Federelements 13 entgegenwirkende Druckkraft D nicht wieder nach oben zurück in die den Strömungskanal 3 verschließende Position drücken.

Die Abbildungen Fig. 7a bis 7c zeigen eine Zwischenposition zwischen den Darstellungen gemäß Fig. 6a bis 6c einerseits und Fig. 8a bis 8c andererseits. Diese Abbildungen Fig. 7a bis 7c wurden aufgenommen, um den Bewegungsablauf zwischen der Nut 28 und der Rastfläche 27 zum Arretieren des Druckelements 9 besser zu verdeutlichen.

Um das Verdrehen des Druckelements 9 zum Betätigen der Zapfen-Schlitz-Steuerung 19 zu erleichtern und auch eine Betätigung mit nur einem Finger zu ermöglichen, sind in der Außenkontur des proximalen Endes des federbelasteten Druckelements 9 Griffmulden 29 ausgebildet.

Zum erneuten Verschließen des Strömungskanals 3 durch den Ventilkolben 4 muss der Operateur nur das Druckelement 9 wieder um die Längsachse 10 des Ventilgehäuses 2 verdrehen, bis die Nut 28 wieder außer Eingriff mit der Rastfläche 27 tritt. Sobald der Operateur dann keine Druckkraft D mehr auf das Druckelement 9 ausübt, drückt das Federelement 13 das Druckelement 9 und somit auch den Ventilkolben 4 zurück in die den Strömungskanal 3 verschließende Position gemäß Fig. 5b.

Die wie zuvor beschrieben ausgebildeten Drucktastenventile 1 zeichnen sich dadurch aus, dass diese dem Operateur eine einfache und ermüdungsfreie Einhandbedienung ermöglichen, da das Druckelement 9 bzw. der Ventilkolben 4 in einer den Strömungskanal 3 freigebenden Position arretierbar ist und aufgrund Ausbildung der zwei Verschlusskörper 15 und 16 an dem gemeinsamen Ventilkolben 4 sichergestellt wird, dass das Öffnen und Verschließen des Strömungskanals 3 und das Abdichten und Öffnen des Drückergehäuses 12 gegenüber der Umgebungsluft U zwangsweise parallel zueinander erfolgt.

### Bezugszeichenliste

- 1: Drucktastenventil
- 2: Ventilgehäuse
- 3: Strömungskanal
- 4: Ventilkolben
- 5: distales Ende (Ventilgehäuse)
- 6: Anschlussstutzen
- 7: Leitung
- 8: Saugleitung
- 9: Druckelement
- 10: Längsachse
- 11: proximales Ende (Ventilgehäuse)
- 12: Drückergehäuse
- 13: Federelement
- 15: Verschlusskörper
- 16: Verschlusskörper
- 17: Ventilsitz
- 18: Ventilsitz
- 19: Klemmring
- 20: Zuluftöffnung
- 21: Zapfen-Schlitz-Steuerung
- 22: Bajonettverschluss
- 23: Raststift
- 24: Kulissenführung
- 25: Abwinklung
- 26: Betätigungshebel
- 27: Rastfläche
- 28: Nut
- 29: Griffmulde

- A: Absaugrichtung
- D: Druckkraft
- S: Saugstromrichtung
- U: Umgebungsluft

## Patentansprüche

1. Manuell betätigbares medizinisches Drucktastenventil mit einem Ventilgehäuse (2), einem in dem Ventilgehäuse (2) ausgebildeten Strömungskanal (3) sowie mit einem Ventilkolben (4), der zwischen einer den Strömungskanal (3) freigebenden und einer den Strömungskanal (3) verschließenden Position verlagerbar im Ventilgehäuse (2) angeordnet ist, wobei der Ventilkolben (4) mit einem am Ventilgehäuse (2) gelagerten, über mindestens ein Federelement (13) federbelastetes Druckelement (9) in Wirkverbindung steht und der Ventilkolben (4) über das federbelastete Druckelement (9) in die den Strömungskanal (3) verschließende Position vorgespannt ist und das federbelastete Druckelement (9) in mindestens einer Position arretierbar ist, in der der mit dem Druckelement (9) in Wirkverbindung stehende Ventilkolben (4) den Strömungskanal (3) zumindest teilweise freigibt, wobei das federbelastete Druckelement (9) in einem am proximalen Ende (11) des Ventilgehäuses (2) angeordneten Drückergehäuse (12) gelagert ist, wobei das federbelastete Druckelement (9) relativ zum Drückergehäuse (12) um die Längsachse (10) des Ventilgehäuses (2) verdrehbar ist und wobei in Richtung der Längsachse (10) des Ventilgehäuses (2) betrachtet am Ventilkolben (4) mit Abstand zueinander zwei Verschlusskörper (15 und 16) ausgebildet sind, die mit entsprechenden Ventilsitzen (17 und 18) zusammenwirken,
**dadurch gekennzeichnet,**
**dass** das federbelastete Druckelement (9) und das Drückergehäuse (12) über zwei Zapfen-Schlitz-Steuerungen (21) miteinander gekoppelt sind, wobei die zwei Zapfen-Schlitz-Steuerungen (21) als Rastfläche (27) und Nut (28) so ausgebildet sind, dass die Nut (28) am Druckelement (9) ausgebildet ist und die Rastfläche (27) als an der Innenseite des Drückergehäuses (12) angeordnete, sich halbkreisförmig radial nach innen erstreckende Fläche ausgebildet ist.

2. Medizinisches Drucktastenventil nach Anspruch 1, **dadurch gekennzeichnet, dass** das federbelastete Druckelement (9) ausschließlich in der Position arretierbar ist, in der der mit dem Druckelement (9) in Wirkverbindung stehende Ventilkolben (4) den Strömungskanal (3) vollständig freigibt.

3. Medizinisches Drucktastenventil nach Anspruch 1 oder 2, **dadurch gekennzeichnet, dass** das mindestens eine Federelement (13), über das Druckelement (9) federbelastet ist, einstückig mit dem Druckelement (9) oder dem Drückergehäuse (12) ausgebildet ist.

4. Medizinisches Drucktastenventil nach einem der Ansprüche 1 bis 3, **dadurch gekennzeichnet, dass** im Übergangsbereich vom Ventilgehäuse (2) zum Drückergehäuse (12) auf der Außenseite der beiden Gehäuse (2) und (12) ein die beiden Gehäuse (2) und (12) miteinander verbindender elastischer Klemmring (19) angeordnet ist.

5. Medizinisches Drucktastenventil nach einem der Ansprüche 1 bis 4, **dadurch gekennzeichnet, dass** am proximalen Ende (11) des federbelasteten Druckelements (9) ein radial nach außen abstehender Betätigungshebel (26) angeordnet ist.

6. Medizinisches Drucktastenventil nach einem der Ansprüche 1 bis 4, **dadurch gekennzeichnet, dass** in der Außenkontur des proximalen Endes (11) des federbelasteten Druckelements (9) Griffmulden (29) ausgebildet sind.

## Claims

1. Manually actuatable medical push-button valve with a valve housing (2), with a flow channel (3) formed in the valve housing (2), and with a valve piston (4) arranged displaceably in the valve housing (2) between a position releasing the flow channel (3) and a position closing the flow channel (3), wherein the valve piston (4) is operatively connected to a pressure element (9) which is mounted on the' valve housing (2) and which is spring-loaded via at least one spring element (13), and the valve piston (4) is pretensioned via the spring-loaded pressure element (9) into the position closing the flow channel (3), and the spring-loaded pressure element (9) is lockable in at least one position in which the valve piston (4) operatively connected to the pressure element (9) at least partially releases the flow channel (3), wherein the spring-loaded pressure element (9) is mounted in a pusher housing (12) arranged at the proximal end (11) of the valve housing (2), wherein the spring-loaded pressure element (9) is rotatable relative to the pusher housing (12) about the longitudinal axis (10) of the valve housing (2), and wherein, viewed in the direction of the longitudinal axis (10) of the valve housing (2), two closure bodies (15 and 16) are formed at a distance from each other on the valve piston (4) and interact with corresponding valve seats (17 and 18),
**characterized in that**
the spring-loaded pressure element (9) and the pusher housing (12) are coupled to each other via two pin-and-slot controls (21), wherein the two pin-and-slot controls (21) are designed as latching surface (27) and groove (28), such that the groove (28) is formed on the pressure element (9) and the latching surface (27) is designed as a semi-circular radially inwardly extending surface arranged on the inner face of the pusher housing (12) .

2. Medical push-button valve according to Claim 1, **characterized in that** the spring-loaded pressure element (9) is lockable exclusively in the position in which the valve piston (4) operatively connected to the pressure element (9) completely releases the flow channel (3).

3. Medical push-button valve according to Claim 1 or 2, **characterized in that** the at least one spring element (13) is spring-loaded via the pressure element (9) and is designed in one piece with the pressure element (9) or the pusher housing (12).

4. Medical push-button valve according to one of Claims 1 to 3, **characterized in that**, in the transition region from the valve housing (2) to the pusher housing (12), an elastic clamping ring (19) connecting the two housings (2) and (12) to each other is arranged on the outside of the two housings (2) and (12).

5. Medical push-button valve according to one of Claims 1 to 4, **characterized in that** a radially outwardly protruding actuating lever (26) is arranged at the proximal end (11) of the spring-loaded push element (9).

6. Medical push-button valve according to one of Claims 1 to 4, **characterized in that** grip recesses (29) are formed in the outer contour of the proximal end (11) of the spring-loaded pressure element (9).

## Revendications

1. Vanne à bouton-poussoir à usage médical pouvant être commandée manuellement et comprenant un boîtier de vanne (2), un conduit d'écoulement (3) formé dans le boîtier de vanne (2) et un piston de vanne (4) qui est disposé dans le boîtier de vanne (2) de manière déplaçable entre une position de libération du conduit d'écoulement (3) et une position de fermeture du conduit d'écoulement (3), le piston de vanne (4) étant relié fonctionnellement à un élément de pression (9) monté sur le boîtier de vanne (2) et contraint élastiquement par au moins un élément à ressort (13) et le piston de vanne (4) étant précontraint dans la position de fermeture du conduit d'écoulement (3) par le biais de l'élément de pression contraint élastiquement (9) et l'élément de pression contraint élastiquement (9) pouvant être bloqué dans au moins une position dans laquelle le piston de vanne (4), qui est relié fonctionnellement à l'élément de pression (9), libère au moins partiellement le conduit d'écoulement (3), l'élément de pression contraint élastiquement (9) étant monté dans un boîtier à poussoirs (12) disposé à l'extrémité proximale (11) du boîtier de vanne (2), l'élément de pression contraint élastiquement (9) pouvant tourner sur l'axe longitudinal (10) du boîtier de vanne (2) par rapport au boîtier à poussoirs (12) et deux corps de fermeture (15 et 16) étant formés sur le piston de vanne (4) à distance l'un de l'autre, lorsque l'on observe dans la direction de l'axe longitudinal (10) du boîtier de vanne (2), lesquels corps de fermeture coopèrent avec des sièges de vanne correspondants (17 et 18),
**caractérisée en ce que**,
l'élément de pression contraint élastiquement (9) et le boîtier à poussoirs (12) sont accouplés l'un à l'autre par le biais de deux commandes à broche et fente (21), les deux commandes à broche et fente (21) étant conçues comme une surface d'encliquetage (27) et une rainure (28) de sorte que la rainure (28) soit formée au niveau de l'élément de pression (9) et la surface d'encliquetage (27) soit formée comme une surface disposée du côté intérieur du boîtier à poussoirs (12) et s'étendant en demi-cercle radialement vers l'intérieur.

2. Vanne à bouton-poussoir à usage médical selon la revendication 1, **caractérisée en ce que** l'élément de pression contraint élastiquement (9) ne peut être verrouillé que dans la position dans laquelle le piston de vanne (4), qui est relié fonctionnellement à l'élément de pression (9), libère complètement le conduit d'écoulement (3).

3. Vanne à bouton-poussoir à usage médical selon la revendication 1 ou 2, **caractérisée en ce que** l'au moins un élément à ressort (13) est contraint élastiquement par le biais de l'élément de pression (9), est formé d'une seule pièce avec l'élément de pression (9) ou le boîtier à poussoirs (12).

4. Vanne à bouton-poussoir à usage médical selon l'une des revendications 1 à 3, **caractérisée en ce qu'**une bague de serrage élastique (19), reliant les deux boîtiers (2) et (12) l'un à l'autre, est disposée dans la zone de transition allant du boîtier de vanne (2) au boîtier à poussoirs (12) du côté extérieur des deux boîtiers (2) et (12).

5. Vanne à bouton-poussoir à usage médical selon l'une des revendications 1 à 4, **caractérisée en ce qu'**un levier d'actionnement (26) faisant saillie radialement vers l'extérieur est disposé à l'extrémité proximale (11) de l'élément de pression contraint élastiquement (9).

6. Vanne à bouton-poussoir à usage médical selon l'une des revendications 1 à 4, **caractérisée en ce que** des dépressions de préhension (29) sont formées dans le contour extérieur de l'extrémité proximale (11) de l'élément de pression contraint élastiquement (9).
